# EUROPEAN PATENT SPECIFICATION

(11) **EP 1 059 899 B1**
(45) Date of publication and mention of the grant of the patent: **07.07.2004**
(21) Application number: 99906583.2
(22) Date of filing: 01.03.1999
(51) Int. Cl.: A61F 5/00, A61F 13/08, A47G 25/90

(54) **AUXILIARY DEVICE FOR PUTTING ON THERAPEUTIC COMPRESSION GARMENTS, ESPECIALLY TIGHTS, KNEE-LENGTH SOCKS AND FULL-LENGTH STOCKINGS**
HILFSVORRICHTUNG ZUM ANZIEHEN VON THERAPEUTISCHEN KOMPRESSIONSSTRUMPFHOSEN, KNIE- UND HALBSTRÜMPFEN
DISPOSITIF AUXILIAIRE POUR METTRE DES ACCESSOIRES DE COMPRESSION THERAPEUTIQUES, EN PARTICULIER DES COLLANTS, DES MI-BAS ET DES BAS

(30) Priority: 06.03.1998 PL 32520998
(43) Date of publication of application: 20.12.2000
(73) Proprietor: Sawlewicz, Pawel, PL-80-462 Gdansk (PL)
(72) Inventor: Sawlewicz, Pawel, PL-80-462 Gdansk (PL)
(74) Representative: Braun, André
(86) International application number: PCT/PL1999/000005
(87) International publication number: WO 1999/044548

(56) References cited:
- EP-A1- 0 620 999
- US-A- 4 260 083
- US-A- 4 651 909
- US-A- 5 356 057

## Description

The subject of the invention presented is an auxiliary device for putting on therapeutic compression garments, especially tights, knee-length socks and fulllength stockings; particularly ones with a closed toe-tip.

A device helping to put on therapeutic compression garments with an open toe-tip is known. It looks like a sock made from low friction factor fabric. Such a device is put on a leg, then a therapeutic compression garment, e.g. a socking, is pulled up over it, and finally the device is pulled off the leg through the hole in the toepart of the stocking. The purpose of using the device is to lower the friction which is present between the skin and the stocking material, while the compression stocking is being pulled up the leg.

Such an existing device can be used only to help put on therapeutic compression garments with an open toe-tip, since only then can it be taken off the leg. EP-A-620 999 and US-A-5 356 057 show such prior art devices.

The invention presented is an auxiliary device for putting on therapeutic compression garments, especially tights, knee-length socks and full-length stockings. It is made from low friction factor fabric and its characteristic feature is its shape, i.e. of a plane figure with a beneficial catch element in the form of a pocket at its one end; at the opposite end, beneficially, there is a fastening element in the form of a tape, press stud or some other well-known fasteners.

In the variant of the invention execution, the pocket has a press stud or Velcro ®.

Owing to its construction, the device according to the invention makes putting on a therapeutic compression garment easy and painless, even when it is the type with a closed toe-tip.

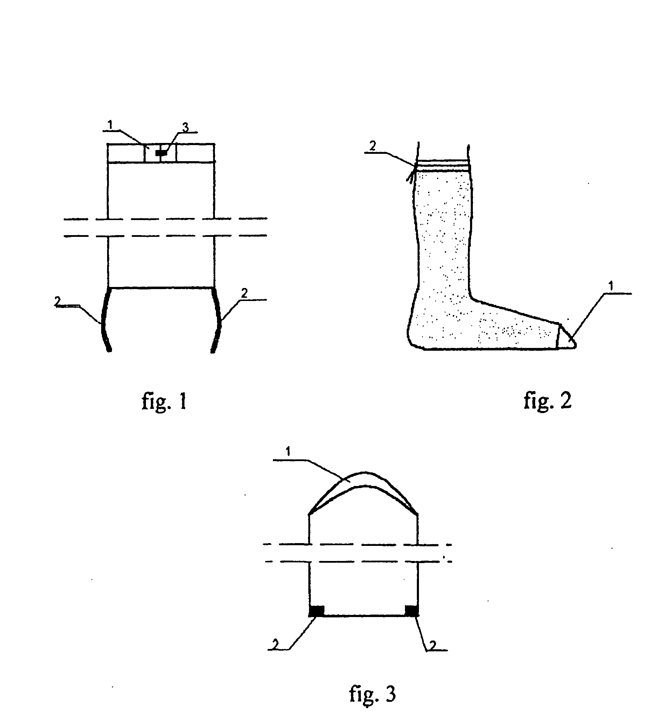

The device according to the invention is depicted in the illustration, where: Fig. 1 presents its schematic view; Fig. 2 shows the device after it has been put on a leg; Fig. 3 shows the view of another version of the device.

### Example I

The device is made from material which is smooth on its both sides and of low friction factor of its surface. The device is rectangular in shape and is 60 centimetres long and 40 centimetres wide. At one end of the device there is a pocket 1, which is 2 centimetres deep. At the opposite end, there is a tape 2 attached. The device is used in the following manner: The toes are inserted into the pocket 1, the cloth of the device is put around the leg so that it covers the whole of the leg surface smoothly, possibly without any folds or wrinkles. The top part of the device is attached to the leg with the tape 2 by tying it around the leg. Once that is done, a compression stocking is pulled up on the leg in a usual way. Then, by using a hand, (through the stocking) the toes are freed from the pocket 1, the tape 2 is undone, and having been pulled by its top the device is removed from under the stocking.

### Example II

The device is made just like in Example I, but the pocket 1 is closed with a fastener 3 in the form of Velcro ®. At the opposite end of the device there is a tape 2. The device is used in the following manner: The toes are inserted into the pocket 1, the cloth of the device is put around the leg so that it covers the whole of the leg surface smoothly, possibly without any folds or wrinkles. The top part of the device is attached to the leg with the tape 2 by tying it around the leg. Once that is done, a compression stocking is pulled up on the leg in a usual way.

Then, the tape 2 is undone, and when the device is energetically pulled at the top, the Velcro ® becomes undone thus freeing the toes from the pocket, and finally the device can be removed from under the stocking by being pulled by its top.

### Example III

The device is made from material which is smooth on its both sides and of low friction factor of its surface. Its pocket 1 is adjusted to the shape of toes. When the device is put on the leg, its shape resembles that of a knee-length sock. There is a fastener 2 at the other end of the device. The device is used in the following manner: After the device has been put on the leg, its top edges are joined with the fastener 2 so that the device will not slide down the leg. Once that is done, a compression stocking is pulled up on the leg in a usual way. Then the fastener 2 is undone, and by using a hand (through the stocking) the toes are freed from the device, which having been pulled by its top, is removed from under the stocking.

## Claims

1. An auxiliary device for putting on therapeutic compression garments, especially tights, knee-length socks and full-length stockings which is made from low friction factor fabric **characterised in that** it has the shape of a plane figure with a beneficial catch element (1) in the form of a pocket for inserting the toes at its one end; at the opposite end there is a fastening element (2) for fastening around the leg in the form of a tape, press stud or some other well-known fasteners.

2. The device described in Claim 1 characterises of the fact that the catch element (1) beneficially has a fastener (3) in the form of a press stud or Velcro ®.

## Patentansprüche

1. Hilfsvorrichtung zum Anziehen von therapeutischen Kompressionsstrumpfhosen, insbesondere Strümpfe, Kniesocken und vollange Strumpfhosen, welche aus einem Gewebe mit niedrigem Reibungskoeffizient hergestellt ist, **dadurch gekennzeichnet, dass** diese Hilfsvorrichtung als flache Form ausgebildet ist, welche an dem einen Ende ein nützliches Auffangelement (1) in Form einer Tasche zum Einführen der Fusszehen enthält und am gegenüber liegenden Ende ein Befestigungselement (2) zur Befestigung um das Bein, in Form eines Bandes, eines Druckknopfes oder eines ändern an sich bekannten Verschlusses, aufweist.

2. Vorrichtung nach Anspruch 1, **dadurch gekennzeichnet, dass** das Auffangelement (1) vorzugsweise einen Verschluss (3) in Form eines Druckknopfes oder Velcro ® umfasst.

## Revendications

1. Dispositif auxiliaire pour mettre des accessoires de compression thérapeutiques, en particulier des collants, des mi-bas et des bas, fabriqué à partir d'un tissu à coefficient de friction faible **caractérisé en ce que** ledit dispositif est une figure plane avec à son extrémité un élément d'accrochage (1) utile sous forme de poche pour insérer les orteils et à son extrémité opposée un élément de fixation (2) pour la fixation autour de la jambe sous forme d'un ruban, d'un bouton-pression ou de toute autre attache connue.

2. Un dispositif selon la revendication 1, **caractérisé en ce que** l'élément d'accrochage (1) comporte de préférence une attache (3) sous forme d'un bouton-pression ou Velcro ®.
